# EUROPEAN PATENT SPECIFICATION

(11) **EP 1 592 447 B1**
(45) Date of publication and mention of the grant of the patent: **28.11.2007**
(21) Application number: 04703839.3
(22) Date of filing: 21.01.2004
(51) Int. Cl.: A61K 41/00, A61K 33/22, A61K 33/00, A61K 31/69, A61K 31/695, A61L 31/06, A61L 31/14, A61P 35/00

(54) **DEVICES AND COMPOSITIONS CONTAINING BORON AND SILICON FOR USE IN NEUTRON CAPTURE THERAPY**
VORRICHTUNGEN UND ZUSAMMENSETZUNGEN MIT BOR UND SILIKON ZUR VERWENDUNG IN DER NEUTRONEN-FANG-THERAPIE
DISPOSITIFS ET COMPOSITIONS CONTENANT DU BORE ET DU SILICIUM A UTILISER DANS UN TRAITEMENT PAR CAPTURE DE NEUTRONS

(30) Priority: 31.01.2003 GB 0302283
(43) Date of publication of application: 09.11.2005
(73) Proprietor: pSiMedica Limited, London EC4M 8SH (GB)
(72) Inventor: CANHAM, Leigh Trevor, Malvern WR14 3SZ (GB)
(74) Representative: Evans, Jacqueline Gail Victoria
(86) International application number: PCT/GB2004/000182
(87) International publication number: WO 2004/067036

(56) References cited:
- WO-A-97/06101
- WO-A-02/067998
- WO-A-03/011251
- WO-A-03/055534
- DE-C- 4 441 483
- GB-A- 2 383 534
- US-A- 5 039 326
- US-B1- 6 299 856
- DATABASE WPI Week 199851, 13 October 1998 (1998-10-13) Derwent Publications Ltd., London, GB; AN 1998-603238 XP002277537 IDOTA TADASHI ET AL.: "Bone metabolic improvement drug and nutritive composition" & JP 10 273442 A (SNOW BRAND MILK PROD CO LTD), 13 October 1998 (1998-10-13)
- SOLOWAY A H ET AL: "THE CHEMISTRY OF NEUTRON CAPTURE THERAPY" CHEMICAL REVIEWS, AMERICAN CHEMICAL SOCIETY. EASTON, US, vol. 98, no. 4, 1 June 1998 (1998-06-01), pages 1515-1562, XP000754987 ISSN: 0009-2665
- CANHAM L ET AL: "WILL A CHIP EVERY DAY KEEP THE DOCTOR AWAY?" PHYSICS WORLD, BRISTOL, GB, vol. 14, no. 7, July 2001 (2001-07), pages 27-31, XP008010482

## Description

This invention relates to new pharmaceutical compositions and their use in treating cancer and other diseases. More specifically the invention relates to new pharmaceutical compositions comprising boron and elemental silicon, and their use in treating cancer.

Cancer remains a significant cause of death, particularly for older members of the population. For example, twenty thousand citizens of the USA contract brain tumours each year, more than a quarter of these being glioblastoma multiform brain tumours, a cancer that is usually fatal within six months of diagnosis.

There are a number of principal methods by which cancer can be treated.

Surgery is ideally a one-time procedure that may cure many types of cancer, particularly in its early stages, or extend the life of a patient. Surgery Inevitably involves hospitalisation of a patient.

Alternatively radiation therapy, which involves the use of high energy X-rays or electron beams to kill cancer cells, can be used to treat cancer. Radiation therapy can be very effective, particularly when the cancer is treated in its early stages and when it is located in a small volume of the patient's body. The high energy radiation can cause damage to healthy tissue. The absorption of a higher dose by the tumour, relative to normal tissue, may be achieved by precise geometric target localization, judicious computer aided treatment planning and accurate beam delivery systems.

A third type of treatment for some cancers is brachytherapy. This is a form of radiation therapy in which the radioactive source is implanted directly into the tumour. An advantage of this technique is that the radiation can be delivered to a confined volume, limiting the effect to the surrounding healthy tissue.
A further potential way of minimising radiation damage to healthy tissue involves the use of boron-10. The technique is known as boron neutron capture therapy (BNCT) and employs thermal and/or epithermal neutrons, which are directed to the region of the tumour. The boron-10, located at the site of the tumour, then interacts with the neutrons to produce highly energetic and cytotoxic lithium-7 and helium-4. The boron-10 and relatively low energy neutrons, each individually, have no significant cytotoxic effect.

The radiation products from BNCT have a short path length of about 12 to 13 microns, which is comparable to a cellular dimensions, and it follows that relatively high concentrations of boron are required in the tumour for the therapy to be effective. It would also be advantageous if the atoms of boron - 10 should be distributed though a substantial part of the tumour. However, provided the interaction of the boron and neutrons only occurs at the site of the tumour, any collateral damage will be relatively minor.

For the BNCT to be effective, between 3 and 30 micrograms of boron-10 per gram of tumour have to be delivered to the affected site. These concentrations are many times those of conventional pharmaceuticals. For example in photodynamic therapy, another 2 step process of treating tumours, the levels of photosensitising drug are typically 100 to 1000 times lower then the levels of boron needed in BNCT. Systemic toxicity is thus a major issue for boronated agents. Such targeting may primarily be accomplished by selectively concentrating boron containing drugs in the tumour.

Research has been conducted into the treatment of primary high grade brain tumours (glioblastoma multiforme) and melanoma. The vehicle used in such treatments was initially boric acid or borax (sodium tetraborate). Other boron agents that have been used for BNCT include sulfhydryl-containing polyhedral borane (BSH) and boronated phenylalanine (BPA).

The results of all phase I (toxicity) and phase II (efficacy) studies have not yet shown a significant advantage for BNCT that would justify phase III trials. A major difficulty is the limited targeting and retention of boronated agents by tumour cells. If agents and techniques could be developed that would allow ten times the concentration of boron in the tumour cells relative to that in normal tissue, BNCT could be used for a much broader range of cancers. Current techniques result in too little boron being localised in the tumour, or too high a concentration in the blood of the patient.

The following papers provide background information for the present invention: Med. Phys. 25 (11), November 1998, p 2220-2225; Radiation Protection Dosimetry Vol 101, Nos 1-4, pp 431-434 (2002); and Radiation Research 151, 235-243 (1999). Each of these papers describes the use of silicon devices in microdosimetry.

Compositions comprising a combination of a boron inorganic or organic compound and a silicon inorganic or organic compound are described in JP 10273442 A. These compositions are described as useful for increasing bone strength. There is no suggestion of their use in the treatment of cancer.

The fabrication of a porous silicon powder from a boron doped polycrystalline silicon germanium alloy for the treatment of cancer by radiotherapy involving localisation of the boron radioisotope is described in WO 02/067998 A.

Boron neutron capture therapy methods are described by Soloway et al. in Chemical reviews, 98(4), pp 1515-1562 (1998).

It is an objective of this invention to address at least some of the above mentioned problems.

According to one aspect, the invention provides a therapeutic composition comprising a silicon component comprising resorbable porous and/or polycrystalline silicon; and a boron component comprising boron-10, the boron component being present at a concentration greater than 10²¹ boron atoms per cm³ of the therapeutic composition.

The silicon component may comprise one or more of microporous silicon, mesoporous silicon, and macroporous silicon.

For the purposes of this specification, microporous silicon contains pores having a diameter less than 20 Å; mesoporous silicon contains pores having a diameter in the range 20 Å to 500 Å; and macroporous silicon contains pores having a diameter greater than 500 Å.

The silicon component may substantially consist of p-type silicon.

For the purposes of this specification resorbable silicon is silicon that is capable, when implanted in a patient, of resorbing at the site of a tumour in a patient. Certain forms of porous and polycrystalline silicon have been found to be bioactive and/or resorbable, as disclosed in PCT/GB96/01863.

For the purposes of the specification a patient may be either a human or an animal.

The boron-10 may form part of a sample of elemental boron and/or may form part of one or more boron compounds.

Boron-10 has an extremely high capture cross section for thermal or epithermal neutrons. A thermal neutron is defined as a neutron having an energy between 1x10⁻⁶ eV and 1 eV and an epithermal neutron is defined as having an energy greater than 1 eV and less than or equal to 10 KeV.

Preferably at least 15% of the boron atoms present in the boron component have a boron-10 nucleus. Naturally occurring boron is 18.7% boron-10 and 81.3% boron-11. More preferably at least 95% of the boron atoms present in the boron component have a boron-10 nucleus. More preferably substantially 100% of the boron atoms present in the boron component have a boron-10 nucleus.

The use of a boron component in combination with a silicon component has several advantages. The doping of silicon with boron is already a very well established process for electronic applications of silicon. Silicon is a material that is resilient to the radiation products from BNCT, is readily processed by semiconductor processing techniques, and certain forms of silicon have favourable biological properties.

The therapeutic composition may comprise at least one implant. The therapeutic composition may comprise at least one silicon implant. The or at least one of the silicon implants may have a largest dimension between 0.1 microns and 2 mm. The or at least one of the silicon implants may have a largest dimension between 0.5 micron and 1 mm. The or at least one of the silicon implants may have a largest dimension between 0.5 microns and 0.1 mm.

The or at least one of the implants may comprise one or more of the following: a seed, a pellet, a bead. The or at least one of the implants may comprise one or more of the following: a staple, a suture, a pin, a plate, a screw, a coil, thread, a nail, and a barb.

The therapeutic composition may comprise a multiplicity of implants. Each implant may comprise part of the silicon component and part of the boron component.

The or at least one of the implants comprises resorbable silicon. The or at least one of the implants may comprise resorbable porous silicon.

The therapeutic composition may form at least part of a single implant.

The therapeutic composition may comprise at least one microparticle. The therapeutic composition may comprise a multiplicity of microparticles. Each microparticle may comprise part of the boron component and part of the silicon component.

Preferably the largest dimension of the or at least one of the microparticles is in the range 0.05 to 5 *µ*m. More preferably the largest dimension of the or at least one of the microparticles is in the range 0.1 to 3 *µ*m. Yet more preferably the largest dimension of the or at least one of the microparticles is in the range 0.15 to 2 *µ*m.

The therapeutic composition may comprise a suspension, suitable for injection into a patient, the suspension comprising a multiplicity of microparticles, each microparticle comprising part of the boron component and part of the silicon component. The suspension may comprise an isotonic solution.

A further advantage of the use of silicon is the availability of semiconductor techniques to fabricate small silicon structures, such as microparticles, microneedles, and/or microbarbs, which are well adapted for treatment of the type of cancer in question.

The boron component is selected from one or more of the following: borax, boric acid, sulfhydryl-containing polyhedral borane (BSH) and boronated phenylalanine (BPA), silicon hexaboride (SiB₆), boron rich porphyrin conjugates, carborane cholesterol analogues, borane salts, nucleosides containing boron clusters, oligonucleotides containing boron clusters, boron anti-body conjugates, boron containing thiouracil derivatives, boronated purine bases, K₂B₁₂H₁₂ and boronated pyridine bases.

The boron may be elemental boron residing as a substitutional impurity in the tetrahedrally coordinated lattice of crystalline silicon. The boron may be elemental boron residing in the grain boundaries of polycrystalline silicon. The silicon component may comprise porous silicon and the boron component may comprise elemental boron that is located in at least some of the pores of the porous silicon or a boron compound that is located in at least some of the pores of the porous silicon.

Preferably the or at least one of the implants comprises silicon and has a shape and composition such that the or at least one of the implants is suitable for brachytherapy.

The silicon component comprises resorbable silicon. More preferably the silicon component comprises resorbable silicon and the boron component is distributed through at least part of the resorbable silicon.

The use of resorbable silicon in combination with a boron component is very advantageous. This is because the resorption of the silicon when placed at the site of the tumour allows the boron component to be released in a form, such as boric acid, which allows the dissemination of the boron through the cancer cells.

If the boron is located in porous silicon, the release rate of the boron may be tuned by altering the porosity and/or pore size of the porous silicon. In general high porosities and high pore sizes have a higher resorption rate than lower pore sizes and low porosities. By tuning the release rate of the boron component, the concentration of boron in the tumour may be maximised relative to the surrounding healthy tumour at the time of neutron irradiation. A further advantage is that the poor solubility of molecular BNCT agents such as BPA may be enhanced by incorporation in porous silicon.

If the boron is in the form of atoms located in the interstices between the silicon atoms of the silicon component, for example as a result of doping the silicon by conventional techniques, then the resorption of the silicon may result in release of the boron in the form of relatively small molecules such as boric acid molecules, thus aiding dispersion into the tumour cells. Further the interaction of the neutrons with the boron and with the surrounding tissue causes some localised heating and this may assist the dissolution of the resorbable silicon.

The silicon component may be micromachined to fabricate one or more implants having a predetermined size and shape, the size and/or shape being chosen to minimise trauma and/or swelling and/or movement of the implant.

The resorbable silicon may comprise derivatised resorbable silicon. The porous silicon may comprise derivatised porous silicon, including the types of derivatised porous silicon disclosed in PCT/US99/01428. Derivatisation may be used to provide targeting of tumour cells. A ligand for cell membrane receptors over-expressed in malignant cells may be covalently linked to the resorbable silicon sub-micron particles.

For the purposes of this specification derivatised porous silicon is defined as porous silicon having a monomolecular, or monatomic layer that is chemically bonded to at least part of the surface, including the surface that defines the pores of the porous silicon. The chemical bonding, between the layer and the silicon, may comprise a Si-C and/or Si-O-C bonding.

Advantageously the therapeutic composition comprises between 0.01 and 100 moles of boron per mole of silicon present in the silicon component. More advantageously the therapeutic composition comprises between 0.1 and 10 moles of boron per mole of silicon present in the silicon component.

Advantageously the therapeutic composition comprises between 0.01 and 100 moles of boron-10 per mole of silicon present in the silicon component. More advantageously the therapeutic composition comprises between 0.1 and 10 moles of boron-10 per mole of silicon present in the silicon component.

The therapeutic composition comprises greater than 10²¹ atoms of boron-10 per cm³ of the composition. The therapeutic composition may comprise between 10²¹ and 3 x 10²² atoms of boron-10 per cm³ of the composition. The therapeutic composition may comprise between 10²¹ and 1.3 x 10²² atoms of boron-10 per cm³ of the composition.

The therapeutic composition may comprise between 1 and 25 atomic percent of boron-10. The therapeutic composition may comprise between 1 and 5 atomic percent of boron-10.

The therapeutic composition may comprise a cytotoxic drug, and the cytotoxic drug may be selected from one or more of: an alkylating agent such as cyclophosphamide, a cytotoxic antibody such as doxorubicin, an antimetabolite such as fluorouracil, a vinca alkaloid such as vinblastine, a hormonal regulator such as GNRH, and a platinum compound such as cis platin.

The therapeutic composition may comprise a water miscible organic solvent. The therapeutic composition may comprise one or more of: methanol, ethanol, 1-propanol, 2-propanol, 1-propen-3-ol (allyl alcohol), 2-methyl-2-propanol (tertiary butyl alcohol), diacotone alcohol, N,N, - dimethylformamide, dimethylsulfoxide, 1,3-dioxane, acetone, pyridine, tetrahydrofuran, ethylene glycol, and propylene glycol.

Preferably the therapeutic composition comprises an ionic carbonate, more preferably the therapeutic composition comprises calcium carbonate and/or sodium carbonate.

Advantageously the therapeutic composition comprises a hydride, more preferably the therapeutic composition comprises a hydride that may be oxidised by contact with a freshly prepared silicon surface.

The therapeutic composition may comprise a gas such as carbon dioxide and/or hydrogen.

Preferably the therapeutic composition comprises a foaming agent.

Carbonates may act as a source of carbon dioxide and hydrides may act as a source of hydrogen once they have been implanted. The release of gas such as carbon dioxide and/or hydrogen may assist the dispersion of the boron component through the tumour.
The dispersion of the boron component may be further assisted by the use of a foaming agent which may interact with the gas to form a foam once the therapeutic composition has been implanted.
According to a further aspect the invention provides the use of a composition comprising a silicon component comprising resorbable porous and/or polycrystalline silicon;and a boron component comprising boron-10; the boron component being present at a concentration greater than 10²¹ boron atoms per cm³ of the therapeutic composition for the manufacture of a medicament for the treatment of cancer by boron neutron capture therapy (BNCT).

The silicon component may comprise one or more of: microporous silicon, mesoporous silicon, and macroporous silicon.

For the purposes of this specification, microporous silicon contains pores having a diameter less than 20 Å; mesoporous silicon contains pores having a diameter in the range 20 Å to 500 Å; and macroporous silicon contains pores having a diameter greater than 500 Å.
The silicon component may substantially consist of p-type silicon.

The boron component is selected from one or more of the following: borax, boric acid, sulfhydryl-containing polyhedral borane (BSH) and boronated phenylalanine (BPA), silicon hexaboride (SiB6), boron rich porphyrin conjugates, carborane cholesterol analogues, borane salts, nucleosides containing boron clusters, oligonucleotides containing boron clusters, boron anti-body conjugates, boron containing thiouracil derivatives, boronated purine bases, K2B12H12 and boronated pyridine bases.

Preferably the treatment further comprises the step of irradiating the patient with thermal and/or epithermal neutrons. Advantageously the treatment comprises the step of irradiating the site or sites at which a cancer is located with thermal or epithermal neutrons.

The therapeutic treatment may be boron neutron capture synovectomy.

The treatment may be used for the treatment of micrometastases, for example for the treatment of micrometastases located in the lungs, bone, the brain, the liver, colon, rectum, breast, ovaries, pancreas, stomach, uterus, testicles, and/or nerve tissues.

The therapeutic treatment may be used to treat malignant conditions including the treatment of immune disorders and to treat rheumatoid or degenerative joint diseases.

The therapeutic treatment may comprise the step of direct intratumoural delivery of the therapeutic composition.

The therapeutic composition may be introduced to the tumour with great precision using a 15variety of techniques.

The therapeutic treatment may comprise the step of using one or more of the following techniques: computer tomography, ultrasound, gamma camera imaging, positron emission tomography, and magnetic resonance tumour imaging.

The boron-10 may be in the form of elemental boron or in the form of a boron compound.

Preferably at least 20% of the boron atoms present in the boron component have a boron-10 nucleus. More preferably at least 95% of the boron atoms present in the boron component have a boron-10 nucleus. Yet more preferably substantially 100% of the boron atoms present in the boron component have a boron-10 nucleus.

Advantageously the therapeutic composition comprises between 0.01 and 100 moles of boron-10 per mole of silicon present in the silicon component. More advantageously the therapeutic composition comprises between 0.1 and 10 moles of boron-10 per mole of silicon present in the silicon component.

The therapeutic composition comprises greater than 10²¹ atoms of boron-10 per cm³ of the composition.. The therapeutic composition may comprise between 10²¹ and 3 x 10²² atoms of boron-10 per cm³ of the composition. The therapeutic composition may comprise between 10²¹ and 1.3 x 10²² atoms of boron-10 per cm³ of the composition.

The therapeutic composition may comprise between 1 and 25 atomic percent of boron-10. The therapeutic composition may comprise between 1 and 5 atomic percent of boron-10.

Advantageously the therapeutic composition comprises at least one implant, the step of introducing the therapeutic composition comprising the step of implanting the or at least one of the implants into the body of a patient. More advantageously the step of implanting the or at least one of the implants comprises the step of biolistically implanting the or at least one of the implants into the site of the tumour.

The step of introducing the therapeutic composition into the patient may comprise the step of introducing the therapeutic composition into the part or parts of a patient's body in which cancer is located.

The step of introducing the therapeutic composition into a patient may comprise the step of introducing the therapeutic composition subcutaneously, intramuscularly, intravascularly, intraperitoneally, and/or epidermally.

The step of introducing the therapeutic composition into a patient may comprise the step of introducing the therapeutic composition into the lungs, bone, the brain, the liver, the colon, the rectum, breasts, ovaries, the pancreas, stomach, the uterus, testicles, and/or nerve tissues.

The step of introducing the therapeutic composition into a patient may comprise the step of introducing the therapeutic composition into tissue consisting of vasculature or a duct.

Preferably the therapeutic composition comprises a multiplicity of microparticles, each microparticle comprising part of the boron component and part of the silicon component. Advantageously the therapeutic product comprises a multiplicity of uniform microparticles each uniform microparticle having a largest dimension in the range 0.05 to 5 *µ*m. More preferably the largest dimension of each uniform microparticle is in the range 0.1 to 3 *µ*m.

Yet more preferably the largest dimension of each uniform microparticle is in the range 0.15 to 2 µm.

The therapeutic composition may comprise at least one implant. The therapeutic composition may comprise at least one silicon implant. The or at least one of the silicon implants may have a largest dimension between 0.1 microns and 2 mm. The or at least one of the silicon implants may have a largest dimension between 0.5 micron and 1 mm. The or at least one of the silicon implants may have a largest dimension between 0.5 microns and 0.1 mm.

The therapeutic composition may comprise a multiplicity of implants, each implant comprising part of the silicon component and part of the boron component.

The therapeutic composition may comprise a multiplicity of boron-silicon implants, each implant comprising part of the silicon component and part of the boron component.

The treatment of a cancer may be by brachytherapy, the implant being implanted into the tumour or into at least one of the tumours.

The step of introducing a therapeutic product into the patient may comprise the step of introducing the therapeutic composition to the site or sites at which the cancer is located.

The step of introducing a therapeutic product into the patient may comprise the step of introducing the therapeutic composition into one or more tumours.

Preferably the therapeutic composition comprises a multiplicity of microparticles suspended in an isotonic solution, and the step of introducing the internal therapeutic composition comprises the step of injecting the suspension into an artery or vein connected to and/or located in organ(s) in which the cancer is located.

The mode of administration of the therapeutic composition will vary with the benign or malignant disorder.

The silicon component comprises resorbable silicon, and the treatment may further comprise the step of introducing the therapeutic composition to the site of the tumour or tumours and allowing the resorbable silicon to resorb.

The silicon component comprises resorbable silicon, and the treatment may further comprise the step of introducing the therapeutic composition to the site of the tumour or tumours and causing the resorption of the resorbable silicon to increase by irradiating the site of the tumour or tumours with thermal or epithermal neutrons.

The boron component may be distributed through part of the resorbable silicon, or the resorbable silicon may form a barrier between the boron component and the site of the tomour or tumours. The arrangement of the resorbable silicon and the boron component may be such that corrosion of the resorbable silicon results in release of the boron component into the tumour.

The therapeutic composition may comprise a solvent, the boron and silicon components being dispersed through the solvent in the form of a suspension. The step of introducing the therapeutic composition into the patient may comprise the step of injecting the suspension into the patient using a needle. The needle may be a flexible skinny needle.

The therapeutic composition may comprise a water miscible organic solvent. The therapeutic composition may comprise one or more of: methanol, ethanol, 1-propanol, 2-propanol, 1-propen-3-ol (allyl alcohol), 2-methyl-2-propanol (tertiary butyl alcohol), diacotone alcohol, N,N, - dimethylformamide, dimethylsulfoxide, 1,3-dioxane, acetone, pyridine, tetrahydrofuran, ethylene glycol, and propylene glycol.

Preferably the therapeutic composition comprises an ionic carbonate, more preferably the therapeutic composition comprises calcium carbonate and/or sodium carbonate.

Advantageously the therapeutic composition comprises a hydride, more preferably the therapeutic composition comprises a hydride that may be oxidised by contact with a freshly prepared silicon surface.

The therapeutic composition may comprise a gas such as carbon dioxide and/or hydrogen.

Preferably the therapeutic composition comprises a foaming agent.

Carbonates may act as a source of carbon dioxide and hydrides may act as a source of hydrogen once they have been introduced into the patient. The release of gas such as carbon dioxide and/or hydrogen may assist the dispersion of the boron component through the tumour. The dispersion of the boron component may be further assisted by the use of a foaming agent which may interact with the gas to form a foam once the therapeutic composition has been implanted.

The therapeutic treatment comprise the step of causing a gas to be released, at the site at which the therapeutic composition has been introduced into the patient. The release of gas in this way may cause the therapeutic composition to be dispersed through the tumour.

The therapeutic treatment may comprise the step of introducing a therapeutic composition, comprising solid carbon dioxide, into a patient.

For the purposes of this specification the term "patient" is either an animal patient or a human patient.

Methods of introducing boron into bulk crystalline silicon are well known. For example traditionally boron is diffused into silicon from gas phase sources such as BBr3, BF3, and B2H6. The first step in all these processes is the formation of boron oxide on the surface of the bulk crystalline silicon. After reduction of the oxide to elemental boron, the boron atoms diffuse into the bulk crystalline silicon. Temperatures at which diffusion typically occurs are usually in the range 900 C to 1400 C.

Boron treated porous silicon may be fabricated by the method comprising the step: (i) incubating a sample of porous silicon in a solution of a boron compound, at a temperature between 20°C and 90°C for a period between 5 minutes and 200 minutes.

The boron compound may be boric acid. The boron compound may be boronated phenylalanine.

Preferably the boron compound solution comprises a solvent selected from: one or more of: ethanol, methanol, and propanol. More preferably the method comprises the further step (ii) of removing the solvent from the porous silicon after the incubation step is complete.

The method of fabricating boron treated porous silicon may further comprise the step of complexing the boron compound with a sugar. The method of fabricating boron treated porous silicon may further comprise the step of complexing the boron compound with a sugar prior to step (i). The method of fabricating boron treated porous silicon may further comprise the step of complexing the boron compound with sucrose.

Advantageously the method of fabricating boron doped porous silicon comprises the step, performed after step (ii), of heating the boron containing porous silicon resulting from step (i) to a temperature of between 800 C and 1500 C for an interval between 5 minutes and 10 hours.

Bulk single crystalline boron doped silicon wafers, that have not been porosified, are commercially available having boron concentrations of less than 8.6 x 10¹⁹ boron atoms per gram of silicon.

Preferably the boron concentration is greater than 5x10²⁰ boron atoms per gram of silicon. More preferably the boron concentration is between 5x10²⁰ and 10²⁴ boron atoms per gram of silicon. Yet more preferably the boron concentration is between 10²¹ and 10²² boron atoms per gram of silicon.

Advantageously the porous silicon has a porosity between 10% and 90%. More advantageously the porous silicon has a porosity between 50% and 80%.

Preferably the porous silicon has a pore size between 2 nm and 500 nm. More preferably the porous silicon has a pore size between 5 nm and 250 nm.

Disclosed is a method of fabricating boron treated porous silicon comprising the step of (i) melting a boron compound, and (ii) allowing the molten boron compound to pass into the pores of the porous silicon.

Advantageously the porous silicon has a porosity between 10% and 90%. More advantageously the porous silicon has a porosity between 50% and 80%.

Preferably the porous silicon has a pore size between 2 nm and 500 nm. More preferably the porous silicon has a pore size between 5 nm and 250 nm.

Therapeutic compositions according to the invention may be fabricated by the method comprising the steps: (a) melting a sample of silicon and a sample of boron, and (b) combining the sample of silicon with the sample of boron so that a molten mixture of boron and silicon is formed, and (c) cooling the molten mixture of boron and silicon.

The step (b) may be performed before or after step (a). The method of fabrication may comprise the further step (d), of atomising the molten mixture of boron and silicon, and cooling the atomized particles to form a boron - silicon particulate product.

The method of fabrication may comprise the further step of stain etching the boron - silicon particulate product to form a porosified particulate product.
Embodiments of the invention will now be described by way of example, with reference to the accompanying drawings, in which:

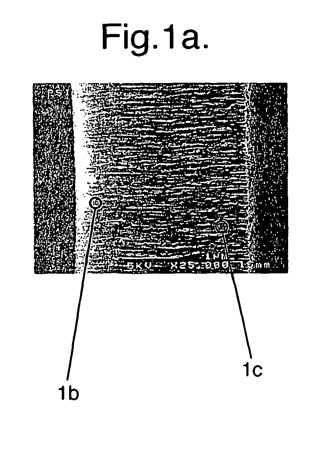

Figure 1a shows an SEM image of a sample of porous silicon that has been treated with boric acid;
Figures 1a and 1b show EDX spectra of the sample measured at the upper and lower portions of the sample porous shown in figure 1a;
Figure 2 shows a SIMS profile for the sample of treated porous silicon shown in figure 1;
Figure 3 shows a SIMS profile for a sample of porous silicon that has been treated with isotopically enriched boric acid;
Figure 4 shows a SIMS profile for a sample of porous silicon that has been treated with boronophenylalanine (BPA) and boric acid; and
Figure 5 shows a SIMS profile for a sample of porous silicon that has been treated with a BPA - sucrose complex.

The therapeutic compositions of the present invention may have a variety of forms suitable for administration by subcutaneous, intramuscular, intravascular, intraperitoneal, or epidermal techniques.

The therapeutic product according to the invention may be spherical, lozenge shaped, rod shaped, in the form of a strip, or cylindrical.

The silicon component may form part of or at least part of: a powder, a suspension, a colloid, an aggregate, and/or a flocculate. The therapeutic composition may comprise an implant or a number of implants, the or each implant comprising the silicon component and the boron component. Such an implant or implants may be implanted into an organ in which a tumour is located in such a manner as to optimise the effect of the anti-cancer component.

### Treatment of a tumour with a suspension

The invention includes a suspension of microparticles, each microparticle comprising a silicon component and a boron component for use in BNCT of a target site in a subject. The microparticles are suspended in an aqueous solution at a selected concentration. The optimal concentration will depend on the characteristics of the microparticles, the type of therapeutic application, and the mode of administration. The aqueous solution may be one or more of water, saline, Ringer's solution, dextrose solution, and 5% human serum albumin. The suspension may be isotonic with the patient's blood. The solution may comprise one or more buffers and/or one or more preservatives and one or more excipients. The solution may comprise polypeptides, proteins, amino acids, carbohydrates, or chelating agents. The suspension of microparticles may be administered via a very narrow diameter needle, a catheter, or by the delivery channel of an endoscope.

### Treatment of a tumour with brachytherapy

Physical accessibility of a tumour to be treated by brachytherapy is essential. Methodology for deep percutaneous access and intratumoral BNCT is similar to that developed for fine needle biopsy techniques. Examples of such techniques include: fine needle aspiration with 20-25 gauge needle, and core needle biopsy in which 11-14 gauge needles are used. Single or multiple injections per tumour may be required. A review of relevant techniques and equipment for this form of administration is given in "Fine needle aspiration of soft tissue lesions" (Clin. Lab. Med. Vol 18, p 507-540 (1998)).

The present invention includes an implant, the implant comprising a boron component and a silicon component. The implant may be used for treatment of a tumour with brachytherapy. The implant may be administered using a needle or trocar (a sharp pointed instrument equipped with a cannula). Placement of the implant can be guided by high-resolution ultrasound or computer tomography.

Ideally methods are used to intraoperatively visualize the needle or trocar insertion into the site of the malignancy. This real-time visualization affords enhanced accuracy of implant placement within the site of the malignancy and allows for the identification and correction of potential sources of error, such as movement of the malignancy and internal tissue distortion that may occur during needle or trocar insertion. This improved imaging ability and use of a needle or trocar obviates the need for surgical incision, permitting this procedure to be done on a cost effective basis.

Alternatively, the organ to undergo the brachytherapy may be surgically debulked and the residual space filled with the therapeutic composition. In another aspect the organ to be treated may be cored with an array of needles and the cores back filled with the therapeutic composition.

### Activation within the tumour

There is optimum time, after introduction of the boron-10, at which neutron radiation is initiated. The calculation of the optimum time is dependent upon a number of factors. Effective BNCT is dependent upon a substantial proportion of the administered boron-10 being retained within the tumour prior to neutron radiation. Not only does the boron-10 have to be at least partly retained in the tumour, but it should be distributed through as much of the tumour as possible. The time taken for these processed to occur may result in neutron irradiation commencing between 30 minutes and 48 hours after the boron-10 has been administered.

Methods for delivering neutron beams of appropriate energy for use in BNCT are described in US 4,516,535. The patient may be positioned in front of the beam of neutrons having energies between 0.5 and 30 KeV. The total dose delivered to the patient may be less than 5 x 10¹² neutrons per cm².

### Preparation of boron containing therapeutic compositions

### Method A

Polycrystalline silicon beads, each bead having a diameter between 0.5 mm and 2 mm and a purity of 99.99 % are jet milled to yield reduced size silicon particles having a diameter between 2 and 6 microns. These reduced size particles are stain etched using HF/HNO₃ acid mixture to yield porous silicon particles. Boron doping to a level of higher than 1 atomic percent may then achieved by incubating the powder in a boric acid/ethanol mixture at a temperature range of 30 to 80 C for between 5 and 200 minutes. Optionally a further thermal drive in step may be employed in which the porous particles are annealed at 600 to 1100 C for 1 to 100 hours, followed by an HF dip.

### Method B

A degenerately doped boron wafer is converted to an array of microneedles by: standard wet etching techniques such as those described in IEEE Transactions in Biomedical Engineering Vol 38, No 8, August 1991, p 758 to 768.

Two sets of deep (200 micron) orthogonal cuts are made into a 380 micron thick wafer. The wafer is rotated by 90 degrees between the first set of n cuts in one direction, and the second set of m cuts in the orthoganal direction. This sawing does not cut through the wafer at any point but creates an array of n x m square columns having an aspect ratio determined by the spacing of the cuts. For a 75 micron wide blade and a pitch of 175 microns one creates 100 micron wide square columns. The subsequent etching processes to define dart-like shapes then have 3 steps. The first chemical etch is to remove saw damage, isotropically reduce the width of the columns and round the edges at the base of the columns. It utilizes an HF: HNO3 etch (eg 5% to 95%) that is conducted with vigorous agitation. The second chemical etch is performed under static conditions that promote preferential attack of the top of the columns to create pointed tips and a tapered shaft. The third etch step is to create a mechanical weakness at the base of the columns which facilitates their detachment from the underlying silicon membrane. This can be achieved by the use of dry etch conditions that undercut the columns.

The narrow neck portions at the base of the needles may be formed by standard deep dry etching techniques in combination with an etch stop (eg silicon oxide).

The needles may be porosified by standard stain etch techniques. The boron concentration may then be increased by bringing the porosified needles into contact with molten boron compound. The molten boron compound is drawn into the pores of the porous silicon needles by surface tension.

When implanted into the site of a tumour, the porous silicon microneedle or microneedles fabricated in this way can release boron both from the boron compound contained in the pores and from the interstitial boron located in the silicon itself.

### Method C

A 0.005 to 0.015 Ωcm boron doped single crystal cz Si wafer is anodised at 35 mA cm⁻² for 90 minutes in an electrolyte comprising equal volumes of 40% HF and ethanol. The applied current density is then raised to 117 mA cm⁻² for 30 seconds. Upon subsequent rinsing in ethanol the entire porous silicon layer detaches from the underlying wafer as a membrane of 65% porosity and thickness of approximately 150 microns. After air drying, the membrane is first crushed manually in filter paper, and then by pestle and mortar. After 1 hour of grinding in the pestle and mortar the average particle size is reduced to less than 50 microns. An alkali buffering agent such as anhydrous borax (Na₂B₄O₇) is then blended with the porous silicon microparticles in dry form.

### Method D

A p-type silicon wafer was anodised in 20% ethanoic HF solution at 32.5 mAcm⁻² for 4 minutes. The silicon wafer was rendered p-type, prior to anodisation, by doping with natural boron, the resistivity of the p-type wafer being less 0.001 ohm cm. Natural boron comprises both boron-10 (18.7%) and boron-11 (81.3%).

The anodisation generated a resorbable mesoprous layer of porous silicon. Segments of the porosified wafer were then immersed in a solution comprising 2.5g boric acid (H₃BO₃) in 25 ml ethanol (Aristar grade BDH) at 70 C for 15 minutes. The boric acid and ethanol solution has a pH of 4. This relatively low pH helps to prevent corrosion of the porous silicon. After removal, the segments were air dried at 100 C without water rinsing.

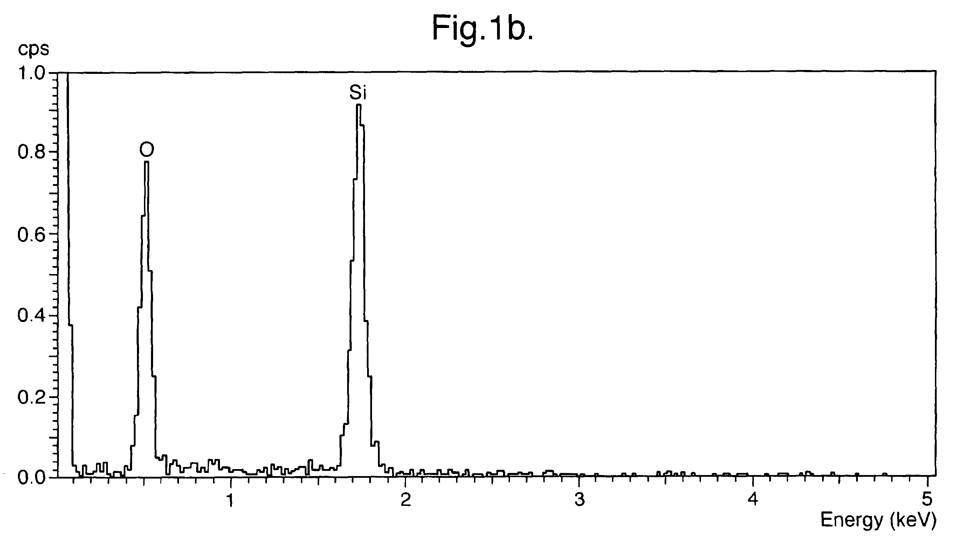

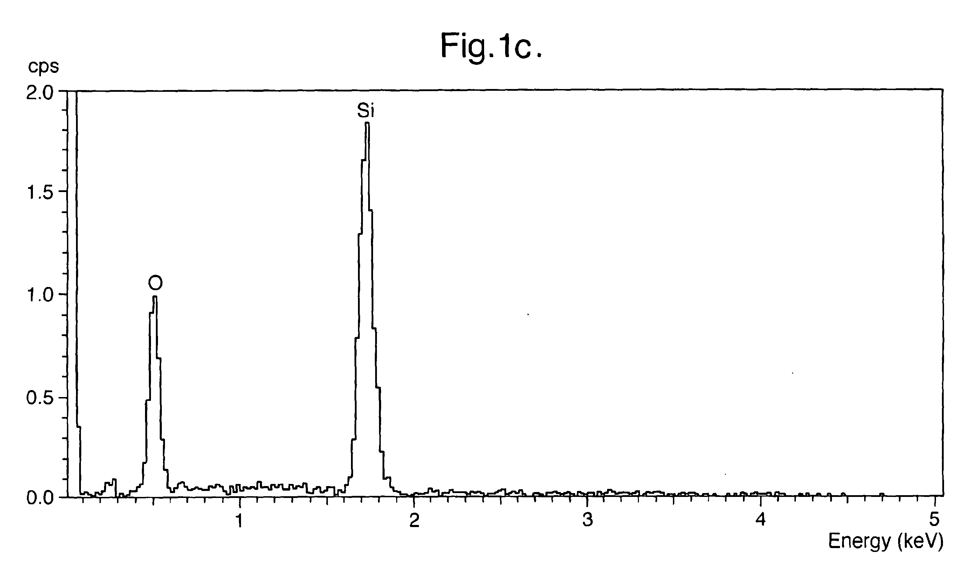

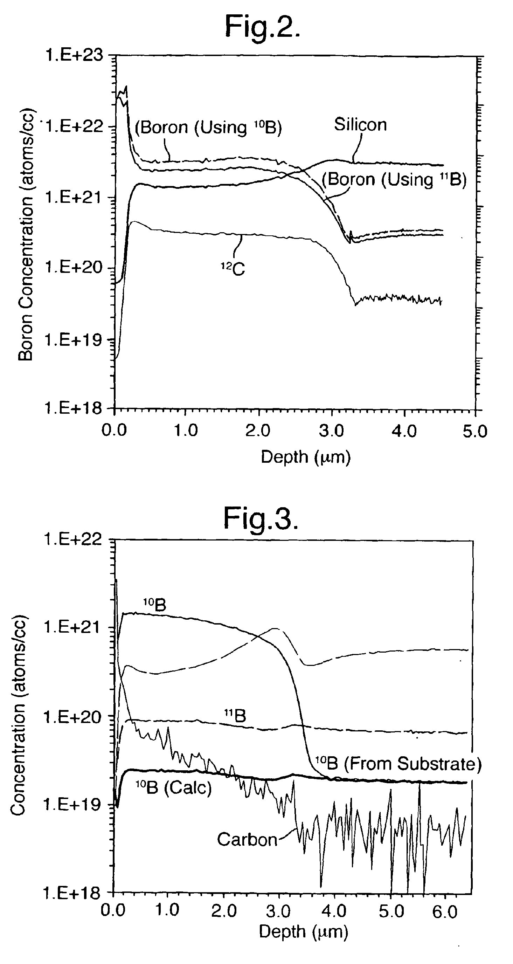

Figure 1a shows an SEM image of the porous silicon, which has been treated with boric acid; figures 1 b and 1 c show EDX spectra of its composition at the upper and lower portions of the porous structure. Figure 1b corresponds to location 1b in figure 1 a. Figure 1c corresponds to location 1c in figure 1a. The EDX spectra indicate that there is an increase in the oxygen content of the porous silicon as a result of the treatment with boric acid. The EDX results are consistent with a boronated oxide coating of the pore walls. The same sample of porous silicon was subjected to Secondary Ion Mass Spectroscopy (SIMS) and the results are shown in figure 2. The figure 2 SIMS plot shows the variation of boron concentration with depth from the surface of the porosified silicon wafer. At depths greater than approximately 3 microns from the surface, corresponding to the bulk crystalline region of the wafer, the boron concentration is approximately 3 x 10²⁰ boron atoms cm⁻³. At lower depths, corresponding to the porous silicon region, the concentration of boron atoms is in the region of 3 x 10²¹ boron atoms cm⁻³. Since natural boric acid was used, the boron-10 concentration in the porous region is 5.6 x 10²⁰ cm⁻³.

### Method E

A p-type silicon wafer was anodised in 20% ethanoic HF solution at 70 mAcm⁻² for 2 minutes, to yield resorbable mesoporous silicon. The silicon wafer was rendered p-type, prior to anodisation, by doping with natural boron.

Segments of the wafer were then immersed in a solution comprising 0.7g isotopically enriched boric acid in 12.5 ml ethanol (Aristar grade BDH) at 60 C for 5 minutes. The isotopically enriched boric acid comprises boron atoms of which 99% are boron-10. The segments were air dried at 100 C without water rinsing.

Figure 3 shows the SIMS profile of the porosified wafer that has been treated with isotopically enriched boric acid. The data reveals a boron-10 concentration of approximately 10²¹ atoms cm⁻³ throughout most of the porous silicon region, which is more than a factor of 10 times higher than the boron-11 concentration. This boron-10 concentration, in the porous silicon region, is equivalent to 2 atomic percent, or 1 boron atom per 50 silicon atoms.

As can be seen from figure 3, the concentration of boron-11 is relatively constant with depth. In other words, it does not vary significantly from the porous to the bulk crystalline region. This shows that the boron-11 content of the porous region is due to natural boron present prior to anodisation.

The level of boron-10 in the porous silicon region, which has been treated with isotopically enriched boric acid, is approximately 100 times greater than the level that would have been present had the porous silicon not been so treated.

### Method F

Boronophenylalanine (BPA) is an FDA approved drug for BNCT. It preferentially accumulates in melanoma cells. Intravenous infusion of BPA is a possible route for targeted delivery to tumours, it has not been widely used due to the drug's poor solubility at physiological pH.

A segment of a porosified silicon wafer, prepared by the process described in method D, was immersed in a suspension of BPA in a boric acid/ethanol/water solution. The boric acid was isotopically enriched (99% of the boron atoms being boron-10) and the BPA comprised natural boron (81.3% of the boron atoms being boron-11). The composition of the solution was: 0.7g boric acid, 0.2g BPA, 12.5 ml ethanol, and 12.5 ml water. The low solubility of the BPA resulted in a supersaturated solution of the compound, having a milky appearance.

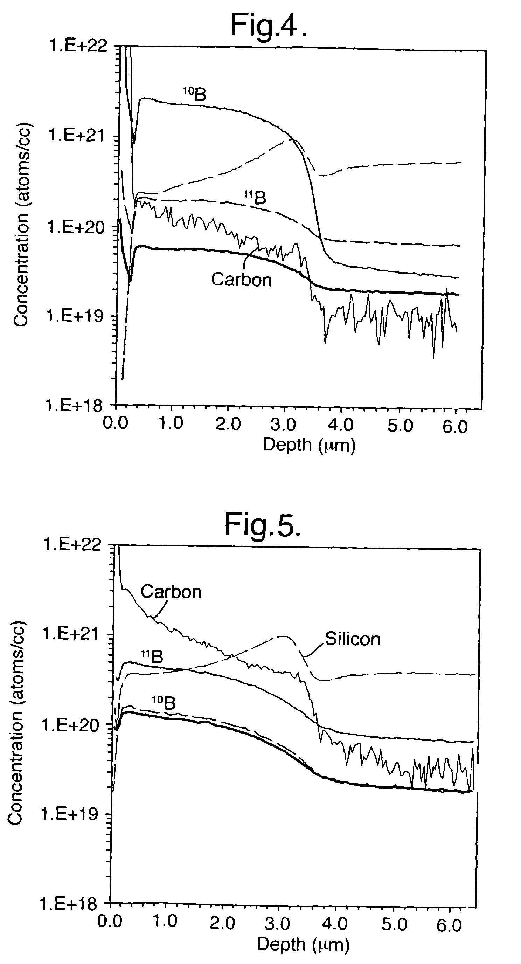

Figure 4 shows a SIMS plot for the porosified segment that had been treated with boric acid and BPA in the manner described above. The boron-11 concentration in the porous region is not significantly increased relative to that of the bulk crystalline substrate. This indicates that very little drug has passed into the pores, despite clear infiltration of the solution phase.

A solution of BPA and boric acid was prepared by a method described in US 5,935,944 and US 6,169,076; 0.2g of BPA was added to 5 ml of deionised water, which results in a milky white suspension. 0.2 g of sucrose and 9 ml of 0.2M NaOH were then added to increase the pH to 10, with the result that a clear solution is obtained. Within 2 minutes the pH is then reduced to 1 by the addition of 1 ml of 5M HCl. The solution, having a concentration of 13 mg/ml of BPA, remained transparent.

Figure 5 shows an SIMS plot for the porosified segment that had been treated with the BPA and sucrose solution prepared above. The prosified segment was incubated in the solution 15 minutes at 50 C. The figure 5 SIMS profile shows elevated levels of both carbon and boron within the porous region as a result of the BPA and sucrose incorporation.

### Method G

A sample of metallurgical grade silicon may be placed in a vitreous carbon crucible on top of a sample of isotopically enriched boric acid having a water content of less than or equal to 170 parts per million. The silicon and boric acid samples may then be melted by induction heating, the temperature of the melt being in excess of 1420 C. In this way a silicon alloy comprising between 0.1 and 1.3 atomic percent boron may be fabricated. A similar process to this is described in US 5,401,331. The molten silicon alloy may be atomised and stain etched to yield solid porous silicon particles comprising boron-10.

### Method H

A film of silicon, comprising boron may be fabricated by vapour deposition. For example silicon and boron hydride gases may be decomposed to yield a silicon film comprising boron and hydrogen, a process that is described in US 4,064,521. In a further example, boron may incorporated in films of crystalline silicon in a non-equilibrium deposition process such as that described in "Understanding ultrahigh doping: the case of boron in silicon" by Luo et al, Physical Review Letters Vol 90(2) p 026103-1 to 4. For example, gas source molecular beam epitaxy has achieved up to 25 atomic percent at 600 C on Si (001) substrates.

Once the film, comprising boron, has been deposited it may be rendered porous and resorbable by standard anodisation techniques. A porous membrane may then be detached from the remainder of the film by increasing the current density for an appropriate interval. The membrane may then be mechanically crushed and filtered to yield microparticles suitable for administration to a patient via a fine gauge needle.

## Claims

1. A therapeutic composition for the treatment of cancer by brachytherapy, the therapeutic composition comprising a silicon component comprising resorbable porous and/or polycrystalline silicon; and a boron component comprising boron-10, the boron component being present at a concentration greater than 10²¹ boron atoms per cm³ of the therapeutic composition.

2. A therapeutic composition according to claim 1 **characterised in that** the boron-10 is in the form of elemental boron.

3. A therapeutic composition according to claim 1 **characterised in that** at least 95% of the boron atoms present in the boron component have a boron-10 nucleus.

4. A therapeutic composition according to claim 1 **characterised in that** the boron component is selected from one or more of the following: borax, boric acid, sulfhydryl-containing polyhedral borane (BSH) and boronated phenylalanine (BPA), silicon hexaboride (SiB₆), boron rich porphyrin conjugates, carborane cholesterol analogues, borane salts, nucleosides containing boron clusters, oligonucleotides containing boron clusters, boron anti-body conjugates, boron containing thiouracil derivatives, boronated purine bases, and boronated pyridine bases.

5. A therapeutic composition according to claim 1 **characterised in that** the boron component comprises boron atoms that are located in the interstices between the silicon atoms of the silicon component.

6. A therapeutic composition according to claim 1 **characterised in that** the boron component is present at a concentration between 10²¹ and 1.3 X 10²², or between 10²¹ and 3 x 10²² boron atoms per cm³ of therapeutic composition .

7. A therapeutic composition according to claim 1 **characterised in that** the silicon component comprises porous silicon and the boron component is located in at least some of the pores of the porous silicon.

8. Use of a composition comprising
a silicon component comprising resorbable porous and/or polycrystalline silicon:
and a boron component comprising boron-10, the boron component being present at a concentration greater than 10²¹ boron atoms per cm³ of the composition, for the manufacture of a medicament for the treatment of cancer by boron neutron capture therapy (BNCT).

9. Use according to claim 8 wherein the boron component is selected from one or more of the following: borax, boric acid, sulfhydryl-containing polyhedral borane (BSH) and boronated phenylalanine (BPA), silicon hexaboride (SiB₆), boron rich porphyrin conjugates, carborane cholesterol analogues, borane salts, nucleosides containing boron clusters, oligonucleotides containing boron clusters, boron anti-body conjugates, boron containing thiouracil derivatives, boronated purine bases, and boronated pyridine bases.

10. Use according to claim 8 wherein the boron-10 is the form of elemental boron.

11. Use according to claim 8 wherein at least 95% of the boron atoms present in the boron component have a boron-10 nucleus.

12. Use according to claim 8 for the treatment of melanoma.

13. Use according to claim 8, wherein the boron component is present at a concentration of between 10²¹ and 10²⁵ boron atoms per cm³ of therapeutic composition.

## Patentansprüche

1. Therapeutische Zusammensetzung zur Behandlung von Krebs durch Brachytherapie, wobei die therapeutische Zusammensetzung eine Siliciumkomponente umfassend resorbierbares poröses und/oder polykristallines Silicium und eine Borkomponente umfassend Bor-10 umfasst, wobei die Borkomponente mit einer Konzentration von größer als 10²¹ Boratomen pro cm³ der therapeutischen Zusammensetzung vorliegt.

2. Therapeutische Zusammensetzung nach Anspruch 1, **dadurch gekennzeichnet, dass** das Bor-10 als elementares Bor vorliegt.

3. Therapeutische Zusammensetzung nach Anspruch 1, **dadurch gekennzeichnet, dass** mindestens 95% der in der Borkomponente vorhandenen Boratome einen Bor-10-Kern aufweisen.

4. Therapeutische Zusammensetzung nach Anspruch 1, **dadurch gekennzeichnet, dass** die Borkomponente ausgewählt ist aus einer oder mehreren der folgenden: Borax, Borsäure, Sulfhydryl enthaltendes polyedrisches Boran (BSH) und boriertes Phenylalanin (BPA), Siliciumhexaborid (SiB₆), Bor-reiche Porphyrin-Konjugate, Carboran-Cholesterol-Analoga, Boransalze, Nucleoside enthaltende Borcluster, Oligonucleotide enthaltende Borcluster, Bor-Antikörper-Konjugate, Bor enthaltende Thiouracil-Derivate, borierte Purinbasen und borierte Pyridinbasen.

5. Therapeutische Zusammensetzung nach Anspruch 1, **dadurch gekennzeichnet, dass** die Borkomponente Boratome umfasst, die sich in den Zwischenräumen zwischen den Siliciumatomen der Siliciumkomponente befinden.

6. Therapeutische Zusammensetzung nach Anspruch 1, **dadurch gekennzeichnet, dass** die Borkomponente mit einer Konzentration zwischen 10²¹ und 1,3 x 10²² oder zwischen 10²¹ und 3 x 10²² Boratomen pro cm³ der therapeutischen Zusammensetzung vorliegt.

7. Therapeutische Zusammensetzung nach Anspruch 1, **dadurch gekennzeichnet, dass** die Siliciumkomponente poröses Silicium umfasst und die Borkomponente sich in mindestens einigen der Poren des porösen Siliciums befindet.

8. Verwendung einer Zusammensetzung umfassend eine Siliciumkomponente umfassend resorbierbares poröses und/oder polykristallines Silicium und eine Borkomponente umfassend Bor-10, wobei die Borkomponente mit einer Konzentration von größer als 10²¹ Boratomen pro cm³ der Zusammensetzung vorliegt, zur Herstellung eines Arzneimittels zur Behandlung von Krebs durch Bor-Neutronen-Einfang-Therapie (BNCT).

9. Verwendung nach Anspruch 8, wobei die Borkomponente ausgewählt ist aus einer oder mehreren der folgenden: Borax, Borsäure, Sulfhydryl enthaltendes polyedrisches Boran (BSH) und boriertes Phenylalanin (BPA), Siliciumhexaborid (SiB₆), Bor-reiche Porphyrin-Konjugate, Carboran-Cholesterol-Analoga, Boransalze, Nucleoside enthaltende Borcluster, Oligonucleotide enthaltende Borcluster, Bor-Antikörper-Konjugate, Bor enthaltende Thiouracil-Derivate, borierte Purinbasen und borierte Pyridinbasen.

10. Verwendung nach Anspruch 8, wobei das Bor-10 als elementares Bor vorliegt.

11. Verwendung nach Anspruch 8, wobei mindestens 95% der in der Borkomponente vorhandenen Boratome einen Bor-10-Kern aufweisen.

12. Verwendung nach Anspruch 8 zur Behandlung von Melanomen.

13. Verwendung nach Anspruch 8, wobei die Borkomponente mit einer Konzentration zwischen 10²¹ und 10²⁵ Boratomen pro cm³ der therapeutischen Zusammensetzung vorliegt.

## Revendications

1. Composition thérapeutique destinée au traitement du cancer par curiethérapie, la composition thérapeutique comprenant un composant contenant du silicium comprenant du silicium résorbable poreux et/ou polycristallin, et un composant contenant du bore comprenant du bore-10, le composant contenant du bore étant présent en une concentration supérieure à 10²¹ atomes de bore par cm³ de la composition thérapeutique.

2. Composition thérapeutique selon la revendication 1 **caractérisée en ce que** le bore-10 est sous la forme de bore élémentaire.

3. Composition thérapeutique selon la revendication 1 **caractérisée en ce qu'**au moins 95 % des atomes de bore présents dans le composant contenant du bore ont un noyau de bore-10.

4. Composition thérapeutique selon la revendication 1 **caractérisée en ce que** le composant contenant du bore est choisi parmi un ou plusieurs de ceux qui suivent : borax, acide borique, borane polyédrique contenant un sulfhydryl (BSH) et phénylalanine borée (BPA), hexaborure de silicium (SiB₆), conjugués de porphyrine riches en bore, analogues du carborane cholestérol, sels de bore, nucléosides contenant des clusters de bore, oligonucléotides contenant des clusters de bore, conjugués de bore et d'anticorps, dérivés de thiouracile contenant du bore, bases puriques borées, et bases pyridiniques borées.

5. Composition thérapeutique selon la revendication 1 **caractérisée en ce que** le composant contenant du bore comprend des atomes de bore qui sont localisés dans les interstices entre les atomes de silicium du composant contenant du silicium.

6. Composition thérapeutique selon la revendication 1 **caractérisée en ce que** le composant contenant du bore est présent en une concentration entre 10²¹ et 1,3 X 10²², ou entre 10²¹ et 3 X 10²² atomes de bore par cm³ de composition thérapeutique.

7. Composition thérapeutique selon la revendication 1 **caractérisée en ce que** le composant contenant du silicium comprend du silicium poreux et le composant contenant du bore est localisé dans au moins une partie des pores du silicium poreux.

8. Utilisation d'une composition comprenant
un composant contenant du silicium comprenant du silicium résorbable poreux et/ou polycristallin ;
et un composant contenant du bore comprenant du bore-10, le composant contenant du bore étant présent en une concentration supérieure à 10²¹ atomes de bore par cm³ de la composition, pour la fabrication d'un médicament destiné au traitement du cancer par boroneutrothérapie (BNCT).

9. Utilisation selon la revendication 8 dans laquelle le composant contenant du bore est choisi parmi un ou plusieurs de ceux qui suivent : borax, acide borique, borane polyédrique contenant un sulfhydryl (BSH) et phénylalanine borée (BPA), hexaborure de silicium (SiB₆), conjugués de porphyrine riches en bore, analogues du carborane cholestérol, sels de bore, nucléosides contenant des clusters de bore, oligonucléotides contenant des clusters de bore, conjugués de bore et d'anticorps, dérivés de thiouracile contenant du bore, bases puriques borées, et bases pyridiniques borées.

10. Utilisation selon la revendication 8 dans laquelle le bore-10 est sous la forme de bore élémentaire.

11. Utilisation selon la revendication 8 dans laquelle au moins 95% des atomes de bore présents dans le composant contenant du bore ont un noyau de bore-10.

12. Utilisation selon la revendication 8 destinée au traitement du mélanome.

13. Utilisation selon la revendication 8, dans laquelle le composant contenant du bore est présent en une concentration entre 10²¹ et 10²⁵ atomes de bore par cm³ de composition thérapeutique.
